(19) Europäisches Patentamt
European Patent Office
Office européen des brevets

(11) **EP 2 630 484 B1**

(12) **FASCICULE DE BREVET EUROPEEN**

(45) Date de publication et mention de la délivrance du brevet:
**15.07.2015 Bulletin 2015/29**

(21) Numéro de dépôt: **11767254.3**

(22) Date de dépôt: **11.10.2011**

(51) Int Cl.:
*G01N 33/06* (2006.01)     *G01N 33/487* (2006.01)

(86) Numéro de dépôt international:
**PCT/EP2011/067689**

(87) Numéro de publication internationale:
**WO 2012/052314 (26.04.2012 Gazette 2012/17)**

(54) **PROCEDE D'EVALUATION DE LA QUANTITE DE METHANE PRODUITE PAR UN RUMINANT LAITIER**

VERFAHREN ZUR BEURTEILUNG DER VON EINEM WIEDERKÄUER PRODUZIERTEN METHANMENGE

METHOD FOR EVALUATING THE QUANTITY OF METHANE PRODUCED BY A DAIRY RUMINANT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.10.2010  FR 1058624**

(43) Date de publication de la demande:
**28.08.2013  Bulletin 2013/35**

(73) Titulaire: **Valorex**
**35210 Combourtille (FR)**

(72) Inventeurs:
• **WEILL, Pierre**
  **F-35770 Vern Sur Seiche (FR)**
• **CHESNEAU, Guillaume**
  **F-35133 Luitre (FR)**

(74) Mandataire: **Regimbeau**
**Parc d'affaires Cap Nord A**
**2, allée Marie Berhaut**
**CS 71104**
**35011 Rennes Cedex (FR)**

(56) Documents cités:
**WO-A1-2009/156453**

• **J. L. ELLIS ET AL: "Evaluation of enteric methane prediction equations for dairy cows used in whole farm models", GLOBAL CHANGE BIOLOGY, vol. 16, no. 12, 20 janvier 2010 (2010-01-20), pages 3246-3256, XP055000433, ISSN: 1354-1013, DOI: 10.1111/j.1365-2486.2010.02188.x**
• **ELLIS J L ET AL: "Modeling methane production from beef cattle using linear and nonlinear approaches", JOURNAL OF ANIMAL SCIENCE, vol. 87, no. 4, 19 décembre 2008 (2008-12-19), pages 1334-1345, XP002619186, AMERICAN SOCIETY OF ANIMAL SCIENCE, US ISSN: 0021-8812, DOI: 10.2527/JAS.2007-0725**

**Description**

La présente invention est relative à un procédé d'évaluation de la quantité de méthane produite par un ruminant laitier.

Le méthane (CH4) entérique est un gaz émis par éructation chez les ruminants. Il se forme pendant la fermentation des aliments dans le rumen de ces animaux et représente une perte d'énergie pour l'animal. Mais le méthane est aussi un puissant gaz à effet de serre.

A l'échelle mondiale, l'élevage contribuerait à hauteur de 18% aux émissions totales de gaz à effet de serre (source FAO, 2006). Le CH4 émis par les fermentations entériques des ruminants représente à lui seul de 3 à 5% de la totalité des émissions mondiales de gaz à effet de serre.

Sa durée de vie dans l'atmosphère est de 12 ans seulement (contre plus de 100 ans pour le gaz carbonique), de sorte que la mise en oeuvre de techniques de réduction des émissions de ce méthane entérique est du plus grand intérêt.

Réduire les émissions de méthane entérique des ruminants répond donc à double objectif, à savoir économique et environnemental.

De nombreuses techniques sont proposées désormais pour réduire les émissions de méthane entérique des ruminants, et notamment des ruminants laitiers.

Cependant, le problème de la mesure de l'efficacité de ces techniques restera posé tant qu'une méthode simple ne sera pas mise au point, qui permettra une mesure routinière.

Si l'émission de méthane entérique est caractéristique de la fermentation des ruminants, les quantités de méthane par kilogramme de lait produit varient fortement en fonction de la productivité des vaches, la nature de la ration, l'écosystème ruminal, etc.

Les données de la littérature scientifique font apparaître à ce sujet une plage de variation par kilogramme de lait extrêmement large. En effet, de 7 à 25 grammes de méthane environ seraient émis par kilogramme de lait produit.

L'« empreinte méthane » du lait peut être définie comme la quantité de méthane émise quotidiennement par une vache en lactation et divisée par le nombre de kilogrammes de lait produit par jour. Elle s'exprime en grammes de méthane (CH4) par kilogramme de lait.

Il est donc intéressant de pouvoir estimer de façon fiable les émissions de méthane de chaque kilogramme de lait en fonction de la composition de celui - ci, afin de pouvoir repérer puis promouvoir des techniques d'élevage de troupeaux qui diminuent et minimisent l'empreinte méthane, c'est-à-dire la quantité de méthane émise pour la production d'un kilogramme de lait.

La littérature scientifique décrit de nombreuses méthodes potentielles de réduction des émissions de méthane entérique.

Sans être exhaustif, on peut noter les méthodes suivantes :

- l'augmentation de la productivité (kilogrammes de lait produit par vache et par jour) augmente les émissions de méthane par vache et par jour, mais réduit la quantité de méthane par litre de lait (le terme « vache » est utilisé ici car il s'agit du ruminant laitier le plus répandu) ;
- l'apport d'huiles alimentaires, qui ne sont pas fermentées dans le rumen ;
- l'usage de substances toxiques pour certaines populations microbiennes du rumen : antibiotiques, huiles essentielles, extraits végétaux, acides gras, etc... ce qui augmente l'usage de l'hydrogène par la voie du propionate au détriment de celle du méthane ;
- l'usage de précurseurs du propionate (malate, fumarate..) qui favorisent aussi la voie du propionate au détriment de la voie du méthane ;
- la combinaison de ces différentes méthodes, etc...

Les publications qui décrivent ces effets utilisent comme moyen de mesure :

a) des techniques In vitro : celles-ci ne sont pas toujours représentatives de l'in vivo.

b) des Techniques In Vivo sur de courtes périodes expérimentales : elles ne présentent pas de garanties de maintien des effets dans la durée. Elles sont toujours difficiles à mettre en oeuvre et présentent des limites expérimentales.

Les plus répandues sont celles dites de la

- « Chambre calorimétrique » et
- « Méthode SF6 », à savoir

la lecture des émissions de méthane en poids, après collecte d'échantillons de gaz éructés, en comparaison à des quantités connues de gaz SF6 issu d'un diffuseur paramétré, introduit dans le rumen de l'animal in vivo.

c) La Mesure au « Détecteur laser » : cette technique récente permettrait de mesurer in situ les émissions de méthane par rayonnement laser, mais les premières publications disponibles (Chagunda & al 2009) ne lui confèrent pas de grande fiabilité.

d) Des prédictions à partir des quantités ingérées ou de la nature de la ration : de nombreuses équations sont proposées par différents auteurs.

Elles sont imprécises et dépendent de nombreux critères qui ne sont pas mesurables en routine, voire pas connus en général (comme l'ingestion des vaches, la fermentescibilité des aliments, etc...)

e) Des prédictions à partir de la productivité des vaches : plus une vache produit de lait et moins les émissions de méthane par kilogramme de lait sont importantes.

Le lien à la productivité n'intègre pas les écarts liés aux types de ration, et une lecture attentive de la bibliographie démontre qu'à niveau de productivité identique, des écarts très importants apparaissent quand les émissions de méthane sont mesurées avec différentes rations.

On décrit dans la demande FR 0854230 au nom du présent demandeur, une méthode fiable et simple qui intègre à la fois la productivité des vaches et l'orientation des fermentations ruminales selon la relation soïchiométrique entre acides gras du rumen, acides gras du lait et méthane.

Des publications récentes (Martin 2008, Chilliard 2009.) confirment ce lien.

Néanmoins, pour mettre en oeuvre cette mesure, il faut :

- connaître la productivité des vaches
- et réaliser un profil en acides gras du lait qui nécessite :

  ◦ une extraction des lipides du lait
  ◦ et une chromatographie en phase gazeuse

La présente invention vise à pallier ces difficultés.

Ainsi, elle propose un procédé d'évaluation de la quantité de méthane produite par un ruminant laitier, caractérisé par le fait qu'il consiste au moins à mesurer la quantité en poids d'au moins un acide gras (AG), issu de la synthèse de novo, dans un échantillon de lait dudit ruminant et à évaluer ladite quantité de méthane selon la relation suivante :

$$CH4 = a*(AG \text{ de Novo}) + y*(AG \text{ de BH}) + z$$

relation dans laquelle :

- « CH4 » est la quantité, en grammes, de méthane produite par kilogramme ou par litre de lait dudit ruminant ;
- « AG de novo » est la quantité mesurée dans l'échantillon, en grammes par kilogramme ou par litre de lait, d'au moins ledit acide gras, prise seule ou combinée à au moins la quantité mesurée d'un autre acide gras de novo ;
- « AG de BH » est la quantité mesurée dans l'échantillon, en grammes par kilogramme ou par litre de lait, d'au moins un acide gras issu de la bio-hydrogénation ruminale, prise seule ou combinée à au moins la quantité mesurée d'un autre acide gras issu de la bio-hydrogénation ruminale ;
- a est compris entre -2 et 2 quand y vaut zéro, ou est compris entre 0.1 et 10 quand y est différent de zéro ;
- y est compris entre -10 et + 10 quand a est différent de zéro, ou est compris entre -50 et -0.1 quand a est égal à zéro ;
- a et y ne pouvant être égaux à zéro simultanément ;
- z est compris entre -100 et + 100.

Grâce à ce procédé, il est possible de s'affranchir de la productivité laitière du ruminant et de déterminer ladite quantité de CH4 par analyse d'un simple échantillon de lait.

Par ailleurs, selon d'autres caractéristiques avantageuses et non limitatives :

- « AG de novo » est choisi parmi les définitions suivantes :

  a) quantité des acides gras saturés (AGS) de 4 à 14 atomes de carbone ;

b) quantité des acides gras saturés de 4 à 16 atomes de carbone ;

c) quantité des acides gras saturés en C12 et C14 ;

d) quantité des acides gras saturés en C4, C6, C8, C10, C12, C14 et C16, pris isolément ou en combinaison d'au moins deux d'entre eux.

- ladite quantité de méthane est donnée par la relation suivante :

$$CH4 = (1,07 \pm 0,5) * \text{Somme des acides gras saturés en C4 à C14} + (4,8 \pm 3).$$

- « AG de BH » est choisi parmi les définitions suivantes :

a) quantité de la totalité des acides gras insaturés (AGI) ;

b) quantité de la totalité des acides gras insaturés (AGI) comportant au moins 18 atomes de carbone ;

c) quantité de la totalité des acides gras insaturés (AGI) comportant au moins 18 atomes de carbone, à l'exception des acide gras insaturés C18 :1 n-9, C18 :2n-6 et C18 :3n-3 ;

d) quantité en acide gras saturé en C18 (C18 :0) ;

e) quantité en acides gras trans ou d'une partie d'entre eux.

- ladite quantité de méthane est donnée par la relation suivante :

$$CH4 = (1,14 \pm 0,4) * \text{Somme des acides gras saturés en C4 à C14} - (0,07 \pm 0,3)* [AGI - (C18 :1n-9 + C18 :2n-6 + C18 :3n-3)] + (4,7 \pm 0,5)$$

dans laquelle « AGI - (C18 :1n-9 + C18 :2n-6 + C18 :3n-3) » représente la quantité de la totalité des acides gras insaturés à l'exception des acides C18 :1 n-9, C18 :2n-6 et C18 :3n-3.

- on mesure ladite quantité d'acide gras issu de la synthèse de novo, par spectroscopie infra-rouge, préférentiellement dans l'infra-rouge moyen.

a) le procédé est répété avec plusieurs échantillons de lait, dits échantillons de référence ;

b) on associe à la quantité mesurée de CH4 de chaque échantillon de référence, son spectre d'absorption infrarouge ;

c) on enregistre le spectre d'absorption infra-rouge d'un nouvel échantillon à tester ;

d) on compare ce spectre aux spectres des échantillons de référence;

e) on déduit la quantité de CH4 associée au nouvel échantillon, par comparaison de son spectre avec ceux des échantillons de référence.

- préférentiellement, à l'étape d), on procède à ladite comparaison par des modèles mathématiques et statistiques et, à l'étape e), on utilise les équations d'évaluation obtenues par les modèles de l'étape d).

D'autres caractéristiques et avantages de l'invention apparaîtront à la lecture de la description détaillée qui va suivre.

Dans l'ensemble de la présente demande, les expressions suivantes sont définies comme suit :

- « lipogenèse de novo » : synthèse d'acide gras par les cellules épithéliales mammaires ;

- « acide gras de novo » : acide gras comportant 16 atomes de carbone ou moins, synthétisés par les cellules épithéliales mammaires ;

- « acide gras de BH » ou « acide gras issu de la bio-hydrogénation ruminale »: acide gras à 18 atomes de carbone ayant subi au moins une hydrogénation lors du processus de fermentation ruminale. Ils présentent zéro, une ou plusieurs insaturations.

Enfin, par le terme « combinée », on entend additionnée ou soustraite ou multipliée ou divisée.

1/ Relation Méthane et AGV (Acides Gras Volatils) - Rumen et méthanogenèse

Le lien entre la production des AGV dans le rumen et la production de méthane est connu et étudié depuis de nombreuses années.

Ainsi, il a été montré que la production d'acétate et de butyrate dans le rumen libère de l'hydrogène et favorise donc la production de méthane, alors que la production de propionate permet l'utilisation de l'hydrogène et donc limite la production de méthane. Cela peut être illustré par les relations suivantes :

$$1 \text{ Glucose (C6) donne 2 Pyruvates (C3) [+ 4H]}$$

$$1 \text{ Pyruvate (C3)} + H_2O = 1 \text{ Acétate} + CO_2 \text{ [+ 2 H]}$$

et

$$1 \text{ Pyruvate} = 1 \text{ propionate (C3) [- 4 H]}$$

Une équation de prédiction a donc été mise au point pour prédire la production de $CH_4$ à partir de la production d'AGV, selon une publication de Moss et al., 2000. Ainsi, plus les fermentations du rumen produisent de C2 (acétate) et de C4 (butyrate), et plus la production de $CH_4$ est importante.

A l'inverse, plus les fermentations du rumen produisent de C3 (propionate), et plus la production de $CH_4$ est diminuée.

L'équation de synthèse qui en découle est définie comme suit (Formule de Moss) : [CH4] = 0,45 [acétate] + 0,40 [Butyrate] - 0,275 [Propionate]

où [x] = quantité de x, en % des AGV totaux.

Le lien entre la lipogenèse de novo et la méthanogenèse apparaît ici puisque C2 et C4 sont les précurseurs des acides gras de novo (AG de novo) synthétisés dans la mamelle.

Plus il y aura de C2 et de C4 produits par les fermentations du rumen et plus il y aura de substrats disponibles pour la synthèse de novo des acides gras du lait.

2/ Mamelle et lipogenèse

Les AGV en C2 et C4 issus des fermentations ruminales sont ensuite prélevés par les cellules épithéliales mammaires pour servir de substrat aux synthèses d'AG de novo.

Si la disponibilité en substrats AGV est le facteur limitant de ces synthèses, (ce qui est très généralement le cas), alors il existe une relation de proportionnalité stricte entre les émissions de CH4 entérique et les excrétions d'AG issus de la synthèse de novo dans le lait, ainsi que le montre le tableau ci-dessous.

| Substrat | AG de novo |
|---|---|
| 2 C2 (ou un C4) font | C4 :0 |
| 3 C2 | C6 :0 |
| 4 C2 | C8 :0 |
| 5 C2 | C10 :0 |
| 6 C2 | C12 :0 |
| 7 C2 | C14 :0 |
| 8 C2 | C16 :0 |

La synthèse des AG de novo dans les cellules endothéliales mammaires se fait presque exclusivement à partir des substrats C2 et C4 issus des fermentations ruminales avec une « empreinte méthane », telle que le suggère la formule de Moss développée ci-dessus.

Elle aboutit alors à la synthèse des acides gras saturés et pairs suivants : C4 :0, C6 :0, C8 :0, C10 :0, C12 :0, C14 :0, C16 :0.

On notera toutefois que, dans un second temps, certains de ces AG saturés (AGS) issus de la synthèse de novo peuvent être désaturés.

Il semble alors tentant d'utiliser la double relation (C2+C4) et CH4 (méthane) d'une part et (C2 + C4) comme précurseurs de la synthèse des AG saturés de novo dans la mamelle pour prédire les quantités de méthane émises à partir des quantités d'AG de novo synthétisées.

Les lipides du lait contiennent également des AG saturés impairs issus d'une synthèse de novo qui utilise les substrats d'AGV pairs (à impact méthane positif), mais aussi du C3 dont l'impact sur la méthanogenèse est négatif. Ainsi pour les AG impairs, le lien entre leurs poids dans le lait et leur « empreinte méthane » sera plus faible.

Cependant, certains AG saturés pairs peuvent avoir une origine exogène, notamment le C16 :0 qui représente à lui seul près du tiers de tous les AG du lait et qui peut provenir de diverses origines, par exemple la mobilisation des réserves du tissu adipeux ou une huile végétale exogène (huile de palme par exemple), etc.

Ainsi pour construire une relation fiable entre l'empreinte méthane par kilogramme de lait et le poids des AG, il faut d'abord disposer d'une mesure fiable du poids d'AG de novo dans chaque kilogramme de lait.

3/ Estimation de la quantité des AG de novo présents dans le lait.

Les mécanismes de synthèse puis d'estérification des AG saturés pairs dans la mamelle comportent de nombreuses voies communes.

Aussi, il est logique de trouver des relations fortes entre les AG saturés pairs du lait. Le tableau suivant (Moate et al, 2007) donne les relations statistiques entre AG saturés pairs de C4 à C16 du lait après une méta-analyse des publications sur ce thème :

| AG | C4 :0 | C6 :0 | C8 :0 | C10 :0 | C12 :0 | C14 :0 |
|---|---|---|---|---|---|---|
| C4 :0 | | | | | | |
| C6 :0 | 0,8* | | | | | |
| C8 :0 | 0,67* | 0,95* | | | | |
| C10 :0 | 0,56* | 0,87* | 0,98* | | | |
| C12 :0 | 0,49* | 0,85* | 0,91* | 0,97* | | |
| C14 :0 | 0,56* | 0,86* | 0,89* | 0,93* | 0,95* | |
| C16 :0 | 0,58* | 0,74* | 0,72* | 0,70* | 0,76* | 0,85* |
| (* $p<0,05$) | | | | | | |

On voit bien que tous ces AG étant liés entre eux par des voies de synthèse en larges parties communes, leurs taux dans le lait sont très corrélés entre eux.

L'acide gras C16 :0 apparaît moins corrélé aux AGS à chaîne courte et moyenne que les autres AG pairs de plus faible longueur de chaîne, peut être à cause de son origine partiellement exogène.

L'acide gras C4 :0 apparaît également moins bien corrélé aux autres AG « de novo », peut-être à cause de voies de synthèse et d'estérification (sur les triglycérides du lait) différentes des autres AG, peut-être aussi à cause de difficultés analytiques.

Ainsi, il apparaît équivalent d'utiliser comme marqueur de la synthèse de novo avec, comme substrat C2 et C4, la somme ou la combinaison de chacun de ces AG.

De façon préférentielle, la somme en poids de tous les AG à chaine courte et moyenne (de C4:0 à C14:0) qui exclut le C16:0 (possiblement d'origine exogène) semble être la relation la plus fiable.

Néanmoins, tout AG pair de C4 à C16, pris isolément, ou toute somme ou combinaison d'au moins deux de ces AG donne une estimation plus ou moins fiable de l'utilisation de substrats C2 et C4 pour la synthèse de novo, donc de la méthanogenèse ruminale.

Ainsi, pour déterminer la quantité de CH4 rejetée, en grammes par kilogramme de lait ou par litre de lait, on utilise l'équation suivante :

$$CH4\ (gr/kg)=\ a^*\ AG\ de\ novo\ +\ z$$

AG de novo : en poids (g/kg de lait),
équation dans laquelle a est compris entre -2 et +2 et z compris entre -100 et +100.

Les AG de novo peuvent être estimés préférentiellement par la teneur en AG saturés du lait (AGS) de 4 à 14 atomes de carbones

Cette formule sous estime la quantité de substrats C2 et C4 utilisés pour la synthèse d'AG mono insaturés de 4 à 16 atomes de carbone après une étape de désaturation mammaire, mais surestime légèrement la part de C2 et C4 dans les AG saturés impairs.

De manière particulièrement préférée, cette quantité est estimée selon l'équation :

$$CH4\ (gr/kg)= 1{,}07 * \text{Somme des AGS de C4 à C14} + 4{,}8$$

Dans laquelle la somme des AGS de C4 à C16 est exprimée en grammes par kilogramme de lait.

Mais il serait possible également d'utiliser d'autres formules qui comprendraient comme variante, à la place de la somme des AGS de C4 à C14 :

- la somme de tous les AGS : il s'agit toutefois d'un paramètre moins précis, car les AGS contiennent aussi des AG exogènes et du C18 :0 issu des biohydrogénations ruminales.
- la somme des AG saturés de 4 à 16 atomes de carbone : une partie non toujours connue du C16 :0 est toutefois d'origine exogène.
- la somme de C12 :0 et C14 :0 ;
- C4:0, C6:0, C8:0, C10:0, C12:0, C14:0, C16:0, pris isolément ou en combinaison, d'au moins deux d'entre eux.

4/ Gain de précision

Le procédé selon l'invention prend bien en compte le lien entre méthanogenèse et lipogenèse tel que décrit plus haut.

Toutefois, les variations des émissions de méthane sont liées ici à la répartition / compétition de l'hydrogène entre les voies de synthèse du méthane et celles de synthèse du propionate.

Or, d'autres voies que celle des méthanogènes et du propionate existent pour l'usage de l'hydrogène. La plupart sont marginales, mais l'hydrogène métabolique produit lors des fermentations anaérobies du rumen peut aussi être utilisé lors des réactions d'hydrogénation des AG poly-insaturés de la ration.

Ainsi l'équation indiquée plus haut peut aussi s'écrire :

$$CH4\ (gr/kg)= a * \text{AG de novo} + y\ \text{AG de BH*} + z$$

**[0062]** * AG de BH = AG issus de la biohydrogénation ruminale Equation dans laquelle :

- a est compris entre - 2 et 2 quand y vaut zéro, ou est compris entre 0.1 et 10 quand y est différent de zéro ;
- y est compris entre - 10 et + 10 quand a est différent de zéro, ou est compris entre - 50 et -0.1 quand a est égal à zéro ;
- a et y ne pouvant être égaux à zéro simultanément ;
- z est compris entre - 100 et + 100.

Les AG issus de la biohydrogénation représentent la somme de tous les AG à 18 et plus atomes de carbone, moins ceux d'origine exogène.

Ils comportent :

- l'acide stéarique (C18 :0), mais celui peut aussi avoir une origine exogène ;
- l'acide oléique (C18 :1 n-9) quand celui ci est issu de la désaturation dans la mamelle du C18:0, mais celui-ci peut avoir aussi une origine exogène.
- tous les AG à plus de 2 insaturations à l'exception des acides linoléique (C18:2 n-6) et alpha-linolénique (C18 :3 n-3) d'origine exogène
- Il conviendra aussi de retirer les AG : C20 :4 n-6, C20:5 n-3, C22:6 n-3, ou à plus grande longueur de chaîne.

Leur contribution spécifique (en dehors des effets généraux des huiles sur la fermentation ruminale appréhendée à partir des AG de novo) à la réduction des émissions de méthane par compétition avec l'hydrogène n'est pas tout à fait négligeable.

A titre d'exemple.

- 360 g de C18 :1 n-9 alimentaire (2% de la ration), hydrogéné à 85 % dans le rumen « consomme » l'hydrogène nécessaire à la synthèse de 0,4 g de CH4 par kilogramme de lait. (≈ 2% des émissions CH4)
- 360 g de C18 :2 n-6 alimentaire, hydrogéné à 85 % dans le rumen « consomme » l'hydrogène nécessaire à la synthèse de 0,7 g de CH4 par kilogramme de lait. (≈ 4%)
- 360 g de C18 :3 n-3 alimentaire, hydrogéné à 85 % dans le rumen « consomme » l'hydrogène nécessaire à la synthèse de 1,1 g de CH4 par kilogramme de lait. (≈ 6%)

La nature des AG issus de la biohydrogénation est complexe.

Parmi les AG à 18 atomes de carbone et plus, certains comme le C18 :1 n-9 peuvent avoir une origine endogène (hydrogénation en C18 :0, puis désaturation en C18 :1 n-9) ou exogène (avec des huiles de colza par exemple).

La valeur de [AGI - (C18 :1 n-9) - (C18 :2 n-6) - (C18 :3 n-3)] est un bon indicateur de La quantité d'AG issus spécifiquement de l'hydrogénation.

Ainsi, l'équation précitée peut avantageusement s'écrire :

CH4 (gr/kg)= 1,14 * (Somme des AGS de C4 à C14)

- 0,07 * [AGI - (C18:1 n-9 + C18:2 n-6 + C18:3 n-3)] + 4,7

Il est possible également d'utiliser d'autres formules qui comprendraient comme variante, à la place de AGI - (C18 :1 n-9 + C18 :2 n-6 + C18 :3 n-3) d'autres indicateurs de la biohydrogénation comme

- la somme des AGI ;
- le C18 :0 ;
- la somme des AG trans ou certains d'entre eux.

Comme indiqué plus haut, l'état de l'art ne permet pas de lecture directe, rapide et facile à mettre en oeuvre. Les prévisions les plus précises nécessitent à ce jour de connaître a minima la teneur en lipides du lait et la productivité des vaches, deux facteurs qui ne sont pas connus en routine.

Le présent procédé permet d'estimer les émissions de méthane par kilogramme de lait à partir d'un seul critère (et non plus un minimum de trois) : la teneur de ce lait en un plusieurs acides gras exprimé en grammes par kilogramme.

5/ Mise en oeuvre du procédé

Depuis quelques années se développe la technique de mesure rapide des AG du lait en grammes par kilogramme de lait (ou par litre) par spectroscopie Infra Rouge.

A partir d'une base de données d'échantillons de lait, cela consiste à mettre en relation pour chaque échantillon de lait, la composition en AG (en grammes par litre) obtenue par une analyse de référence (telle que la chromatographie en phase gazeuse) et le spectre d'absorption lumineuse obtenu par analyse infrarouge. Par des méthodes mathématiques et statistiques faisant usage d'équations (ou de calibrations), dès lors que le niveau de fiabilité de la mesure est satisfaisant, il devient alors possible d'estimer par des équations les AG de tout échantillon de lait par analyse infrarouge, en utilisant les équations d'estimation déterminées préalablement.

Puisque le lien stoïchiométrique entre AG du lait et l'émission de méthane est connu, il est tentant d'utiliser la lecture directe des AG du lait en grammes par kilogramme (ou par litre) pour évaluer « l'empreinte méthane » du lait.

Il n'y a plus besoin de connaître la productivité par vache, ni la teneur en lipides totaux du lait.

L'usage de la spectroscopie infra-rouge, plus précisément en moyen infra-rouge permet de mesurer la teneur en AG d'échantillons de lait liquide directement en grammes / kilogramme de lait (ou par litre).

La précision de cette méthode est de plus en plus fiable, ainsi que l'indiquent des publications récentes (Soyeurt et al, 2010).

Cette avancée de la technique permet de mettre en oeuvre la présente invention avec une grande facilité.

Il n'est donc plus nécessaire de réaliser une extraction des lipides et une chromatographie en phase gazeuse, ni de connaître la teneur en lipides totaux du lait et la productivité des vaches.

Selon l'invention, la mesure de l'empreinte méthane de chaque kilogramme de lait peut être réalisée, de manière routinière, à chaque instant.

Au lieu de mesurer successivement les AG, pris isolément ou en combinaison, par l'analyse spectrale infrarouge du lait, puis le CH4 à partir de ces AG, il est également envisageable de déduire directement le CH4 à partir de l'analyse spectrale infrarouge.

En effet, les quantités d'AG de novo, a minima, sont aujourd'hui très bien déterminées dans le moyen infrarouge et sont spécifiquement rattachés à des bandes spectrales d'absorption identifiées, et dont les longueurs d'onde correspondent à leur absorption lumineuse. Il est donc possible de déterminer directement le CH4 d'un échantillon de lait à partir d'une analyse spectrale sans passer par la mesure des AG du lait, mais en utilisant les mêmes bandes spectrales.

**Revendications**

1. Procédé d'évaluation de la quantité de méthane produite par un ruminant laitier, **caractérisé par le fait qu'**il consiste au moins à mesurer la quantité en poids d'au moins un acide gras (AG), issu de la synthèse de novo, dans un échantillon de lait dudit ruminant et à évaluer ladite quantité de méthane selon la relation suivante :

$$CH4 = a*(AG\ de\ Novo) + y*(AG\ de\ BH) + z$$

relation dans laquelle :

- « CH4 » est la quantité, en grammes, de méthane produite par kilogramme ou par litre de lait dudit ruminant ;
- « AG de novo » est la quantité mesurée dans l'échantillon, en grammes par kilogramme ou par litre de lait, d'au moins ledit acide gras, prise seule ou combinée à au moins la quantité mesurée d'un autre acide gras de novo ;
- « AG de BH » est la quantité mesurée dans l'échantillon, en grammes par kilogramme ou par litre de lait, d'au moins un acide gras issu de la bio-hydrogénation ruminale, prise seule ou combinée à au moins la quantité mesurée d'un autre acide gras issu de la bio-hydrogénation ruminale ;
- a est compris entre -2 et 2 quand y vaut zéro, ou est compris entre 0.1 et 10 quand y est différent de zéro ;
- y est compris entre -10 et + 10 quand a est différent de zéro, ou est compris entre - 50 et - 0.1 quand a est égal à zéro ;
- a et y ne pouvant être égaux à zéro simultanément ;
- z est compris entre -100 et + 100.

2. Procédé selon la revendication 1, **caractérisé par le fait que** « AG de novo » est choisi parmi les définitions suivantes :

a) quantité des acides gras saturés (AGS) de 4 à 14 atomes de carbone ;
b) quantité des acides gras saturés de 4 à 16 atomes de carbone ;
c) quantité des acides gras saturés en C12 et C14 ;
d) quantité des acides gras saturés en C4, C6, C8, C10, C12, C14 et C16, pris isolément ou en combinaison d'au moins deux d'entre eux.

3. Procédé selon la revendication 2, dans lequel y vaut zéro, **caractérisé par le fait que** ladite quantité de méthane est donnée par la relation suivante :

$$CH4\ =\ (1,07 \pm 0,5) *\ Somme\ des\ acides\ gras\ saturés\ en\ C4\ à\ C14\ +$$
$$(4,8 \pm 3).$$

4. Procédé selon l'une des revendications 1 ou 2, dans lequel y est différent de zéro, **caractérisé par le fait que** « AG de BH » est choisi parmi les définitions suivantes :

a) quantité de la totalité des acides gras insaturés (AGI) ;
b) quantité de la totalité des acides gras insaturés (AGI) comportant au moins 18 atomes de carbone ;
c) quantité de la totalité des acides gras insaturés (AGI) comportant au moins 18 atomes de carbone, à l'exception des acide gras insaturés C18 :1 n-9, C18 :2n-6 et C18 :3n-3 ;
d) quantité en acide gras saturé en C18 (C18 :0) ;
e) quantité en acides gras trans ou d'une partie d'entre eux.

5. Procédé selon la revendication 4, **caractérisé par le fait que** ladite quantité de méthane est donnée par la relation suivante :

$$CH4 = (1,14\pm 0,4) * \text{Somme des acides gras saturés en C4 à C14} - (0,07\pm 0,03) * [AGI - (C18:1n\text{-}9 + C18:2n\text{-}6 + C18:3n\text{-}3)] + (4,7\pm 0,5),$$

dans laquelle « AGI - (C18 :1n-9 + C18 :2n-6 + C18 :3n-3) » représente la quantité de la totalité des acides gras insaturés à l'exception des acides C18 :1 n-9, C18 :2n-6 et C18 :3n-3.

6. Procédé selon l'une des revendications précédentes, **caractérisé par le fait que** l'on mesure ladite quantité d'acide gras issu de la synthèse de novo, par spectroscopie infra-rouge, préférentiellement dans l'infra-rouge moyen.

7. Procédé selon l'une des revendications précédentes, **caractérisé par le fait que** :

a) il est répété avec plusieurs échantillons de lait, dits échantillons de référence ;
b) on associe à la quantité mesurée de CH4 de chaque échantillon de référence, son spectre d'absorption infrarouge ;
c) on enregistre le spectre d'absorption infra-rouge d'un nouvel échantillon à tester ;
d) on compare ce spectre aux spectres des échantillons de référence;
e) on déduit la quantité de CH4 associée au nouvel échantillon, par comparaison de son spectre avec ceux des échantillons de référence.

8. Procédé selon la revendication 7, **caractérisé par le fait que**, à l'étape d), on procède à ladite comparaison par des modèles mathématiques et statistiques et, à l'étape e), on utilise les équations d'évaluation obtenues par les modèles de l'étape d).

**Patentansprüche**

1. Verfahren zur Beurteilung der von einem Milchwiederkäuer produzierten Methanmenge, **dadurch gekennzeichnet, dass** es mindestens darin besteht, die Gewichtsmenge mindestens einer Fettsäure (AG) im Ergebnis der de-novo-Synthese in einer Milchprobe des Wiederkäuers zu messen und die Methanmenge gemäß der folgenden Gleichung zu beurteilen:

$$CH4 = a*(AG\ de\ Novo) + y*(AG\ de\ BH) + z$$

wobei in dieser Gleichung:

- "CH4" die von dem Wiederkäuer je Kilogramm oder je Liter Milch produzierte Methanmenge in Gramm ist,
- "AG de novo" die in der Probe in Gramm je Kilogramm oder je Liter Milch gemessene Menge mindestens der Fettsäure ist, die allein oder in Kombination mit mindestens der gemessenen Menge einer anderen de-novo-Fettsäure herangezogen wird,
- "AG de BH" die in der Probe in Gramm je Kilogramm oder je Liter Milch gemessene Menge mindestens einer Fettsäure als Ergebnis der biologischen Wiederkäuer-Hydrierung ist, die allein oder in Kombination mit mindestens der gemessenen Menge einer anderen Fettsäure als Ergebnis der biologischen Wiederkäuer-Hydrierung herangezogen wird,
- a zwischen -2 und 2 inklusive ist, wenn y gleich Null ist, oder zwischen 0,1 und 10 inklusive ist, wenn y ungleich Null ist,
- y zwischen -10 und +10 inklusive ist, wenn a ungleich Null ist, oder zwischen -50 und -0,1 inklusive ist, wenn a gleich Null ist,
- a und y nicht gleichzeitig gleich Null sein können,
- z zwischen -100 und +100 inklusive ist.

2. Verfahren nach Anspruch 1, **dadurch gekennzeichnet, dass** "AG de novo" aus den folgenden Definitionen ausgewählt ist:

a) Menge der gesättigten Fettsäuren (AGS) mit 4 bis 14 Kohlenstoffatomen,
b) Menge der gesättigten Fettsäuren mit 4 bis 16 Kohlenstoffatomen,
c) Menge der gesättigten $C_{12}$- und $C_{14}$-Fettsäuren,
d) Menge der gesättigten $C_4$-, $C_6$-, $C_8$-, $C_{10}$-, $C_{12}$-, $C_{14}$- und $C_{16}$-Fettsäuren, die isoliert oder in Kombination von mindestens zwei von ihnen herangezogen werden.

3. Verfahren nach Anspruch 2, wobei y Null ist, **dadurch gekennzeichnet, dass** die Methanmenge durch die folgende Gleichung wiedergegeben wird:

```
CH4 = (1,07 ± 0,5) * Summe der gesättigten C₄- bis
C₁₄-Fettsäuren + (4,8 ± 3).
```

4. Verfahren nach einem der Ansprüche 1 oder 2, wobei y ungleich Null ist, **dadurch gekennzeichnet, dass** "AG de BH" aus den folgenden Definitionen ausgewählt ist:

a) Menge der ungesättigten Fettsäuren (AGI) insgesamt,
b) Menge der ungesättigten Fettsäuren (AGI) insgesamt mit mindestens 18 Kohlenstoffatomen,
c) Menge der ungesättigten Fettsäuren (AGI) insgesamt mit mindestens 18 Kohlenstoffatomen, mit Ausnahme der ungesättigten Fettsäuren C18 : 1n-9, C18 :2n-6 und C18 :3n-3,
d) Menge der gesättigten $C_{18}$-Fettsäuren (C18 :0),
e) Menge der Transfettsäuren oder eines Teils von ihnen.

5. Verfahren nach Anspruch 4, **dadurch gekennzeichnet, dass** die Methanmenge durch die folgende Gleichung wiedergegeben wird:

```
CH4 = (1,14 ± 0,4) * Summe der gesättigten C₄- bis
C₁₄-Fettsäuren - (0,07 ± 0,03) * [AGI - (C18 :1n-9 +
C18 :2n-6 + C18 :3n-3)] + (4,7 ± 0,5),
```

wobei "AGI - (C18 : :1n-9 + C18 :2n-6 + C18 : 3n-3)" die Menge der ungesättigten Fettsäuren insgesamt mit Ausnahme der Säuren C18 :1n-9, C18 :2n-6 und C18 :3n-3 darstellt.

6. Verfahren nach einem der vorangehenden Ansprüche, **dadurch gekennzeichnet, dass** die aus der de-novo-Synthese hervorgegangene Fettsäuremenge durch Infrarotspektroskopie, vorzugsweise im mittleren Infrarotbereich, gemessen wird.

7. Verfahren nach einem der vorangehenden Ansprüche, **dadurch gekennzeichnet, dass**:

a) es mit mehreren als Referenzproben bezeichneten Milchproben wiederholt wird,
b) der gemessenen CH4-Menge jeder Referenzprobe ihr Infrarot-Absorptionsspektrum zugeordnet wird,
c) das Infrarot-Absorptionsspektrum einer neuen zu testenden Probe gespeichert wird,
d) dieses Spektrum mit den Spektren der Referenzproben verglichen wird,
e) die der neuen Probe zugeordnete CH4-Menge durch Vergleich ihres Spektrums mit denen der Referenzproben abgeleitet wird.

8. Verfahren nach Anspruch 7, **dadurch gekennzeichnet, dass** in Schritt d) der Vergleich anhand mathematischer und statistischer Modelle durchgeführt wird und in Schritt e) die Beurteilungsgleichungen im Ergebnis der Modelle von Schritt d) verwendet werden.

**Claims**

1. Method for evaluating the quantity of methane produced by a dairy ruminant, **characterised in that** it involves at least measuring the weight quantity of at least one fatty acid (AG), derived from de novo synthesis, in a sample of milk from said ruminant and evaluating said quantity of methane according to the following formula:

$$CH4 = a*(de\ novo\ AG) + y*(BH\ AG) + z$$

relation wherein:

- "CH4" is the quantity, in grams, of methane produced per kilogram or per litre of milk by said ruminant;
- "de novo AG" is the quantity measured in the sample, in grams per kilogram or per litre of milk, of at least said fatty acid, taken alone or in combination with at least the measured quantity of another de novo fatty acid;
- "BH AG" is the measured quantity in the sample, in grams per kilogram or per litre of milk, of at least one fatty acid derived from ruminal bio-hydrogenation, taken alone or in combination with at least the measured quantity of another fatty acid derived from ruminal bio-hydrogenation;
- a is comprised between - 2 and 2 when y is equal to zero, or is comprised between 0.1 and 10 when y is different to zero;
- y is comprised between - 10 and + 10 when a is different to zero, or is comprised between - 50 and - 0.1 when a is equal to zero;
- a and y not being able to be equal to zero simultaneously;
- z is comprised between - 100 and + 100.

2.  Method according to claim 1, **characterised in that** "de novo AG" is selected from the following definitions:

    a) quantity of saturated fatty acids (AGS) from 4 to 14 carbon atoms;
    b) quantity of saturated fatty acids from 4 to 16 carbon atoms;
    c) quantity of C12 and C14 saturated fatty acids; d)quantity of C4, C6, C8, C10, C12, C14 and C16 saturated fatty acids, taken alone or in combination of at least two thereof.

3.  Method according to claim 2, wherein y is equal to zero, **characterised in that** said quantity of methane is given by the following formula:

$$CH4 = (1.07 \pm 0.5)*\ Sum\ of\ C4\ to\ C14\ saturated$$
$$fatty\ acids + (4.8 \pm 3).$$

4.  Method according to one of claims 1 or 2, wherein y is different to zero, **characterised in that** "BH AG" is selected from the following definitions:

    a) quantity of the totality of unsaturated fatty acids (AGI) ;
    b) quantity of the totality of unsaturated fatty acids (AGI) comprising at least 18 carbon atoms;
    c) quantity of the totality of unsaturated fatty acids (AGI) comprising at least 18 carbon atoms, with the exception of C18 :1 n-9, C18 :2n-6 and C18 :3n-3 unsaturated fatty acids;
    d) quantity of C18 saturated fatty acid (C18 : 0) ;
    e) quantity of trans fatty acids or a part thereof.

5.  Method according to claim 4, **characterised in that** said quantity of methane is given by the following formula:

$$CH4 = (1.14 \pm 0.4)\ *\ Sum\ of\ C4\ to\ C14\ saturated$$
$$fatty\ acids - (0.07 \pm 0.03)\ *\ [AGI - (C18\ :1\ n-9 + C18$$
$$:2n-6 + C18\ :3n-3)] + (4.7 \pm 0.5),$$

wherein "AGI - (C18 :1n-9 + C18 :2n-6 + C18 :3n-3)" represents the quantity of the totality of unsaturated fatty acids with the exception of C18 :1 n-9, C18 :2n-6 and C18 :3n-3 acids.

6.  Method according to one of the preceding claims, **characterised in that** one measures said quantity of fatty acid derived from de novo synthesis, by infrared spectroscopy, preferentially in the mid infrared.

7.  Method according to one of the preceding claims, **characterised in that**:

    a) it is repeated with several samples of milk, known as reference samples;
    b) one associates with the measured quantity of CH4 of each reference sample, the infrared absorption spectrum thereof;
    c) one records the infrared absorption spectrum of a new sample to test;
    d) one compares said spectrum with the spectra of the reference samples;
    e) one deduces the quantity of CH4 associated with the new sample, by comparison of the spectrum thereof with those of the reference samples.

8.  Method according to claim 7, **characterised in that**, at step d), one carries out said comparison by mathematical and statistical models and, at step e), one uses the evaluation equations obtained by the models of step d).

**RÉFÉRENCES CITÉES DANS LA DESCRIPTION**

**Documents brevets cités dans la description**

- FR 0854230